# EUROPEAN PATENT APPLICATION

(11) **EP 4 778 516 A1**
(43) Date of publication of application: **22.07.2026**
(21) Application number: 24865301.6
(22) Date of filing: 02.09.2024
(51) Int. Cl.: A61K 31/085, A61P 13/08, A61P 19/00, A61P 19/10, A61P 29/00, A61P 35/00, A61P 43/00

(54) **CALCIFICATION ENHANCER FOR OSTEOBLAST MC3T3-E1 CELL**

(30) Priority: 15.09.2023 JP 2023150157
(71) Applicant: Watanabe Oyster Laboratory Co., Ltd., Hachioji-Shi Tokyo 192-0154 (JP)
(72) Inventor: WATANABE Mitsugu, Hachioji-shi, Tokyo 192-0154 (JP)
(74) Representative: Plougmann Vingtoft a/s
(86) International application number: PCT/JP2024/031459
(87) International publication number: WO 2025/057799

(57) **Abstract**

[Problem]

The present invention provides a novel strategy for protecting against and treating impaired bone formation and calcification of osteoblasts associated with inflammatory conditions in the bone marrow microenvironment, by providing a suppressing agent or the like including 3,5-dihydroxy-4-methoxybenzyl alcohol (DHMBA) as an active ingredient.

[Solution]

The present invention is characterized by including 3,5-dihydroxy-4-methoxybenzyl alcohol (DHMBA) as an active ingredient and having an action of enhancing calcification of osteoblast MC3T3-E1 cells.

## Description

### TECHNICAL FIELD

The present invention relates to, for example, a calcification enhancer for osteoblast MC3T3-E1 cells.

### BACKGROUND ART

A phenolic antioxidant, 3,5-dihydroxy-4-methoxybenzyl alcohol (DHMBA), which has been discovered in, for example, the Pacific oyster (Crassostrea gigas), functions as a superior peroxyl radical scavenger compared with other strong antioxidants such as Trolox. Accordingly, DHMBA may play an important role in the prevention of health disorders.

In the present invention, it has been elucidated whether DHMBA prevents calcification impairment of mouse osteoblast MC3T3-E1 cells induced under inflammatory conditions by using mouse macrophage RAW264.7 cells in vitro.

In an osteoblast culture system, the addition of DHMBA (1 to 100 mM) did not affect the proliferation or death of MC3T3-E1 cells. Under this condition, DHMBA was found to enhance the calcification of osteoblasts. DHMBA also suppressed the decrease in calcification of MC3T3-E1 cells caused by the addition of the inflammatory cytokine TNF-a.

DHMBA inhibited TNF-a production induced by lipopolysaccharide (LPS) stimulation in cultures of macrophage RAW264.7 cells. Under LPS stimulation, proliferation of MC3T3-E1 cells was suppressed by crosstalk resulting from co-culture with RAW264.7 cells.

Interestingly, it has been found that proliferation of MC3T3-E1 cells is suppressed by culturing the cells with a medium obtained by culturing macrophages with LPS. This effect was suppressed in the presence of DHMBA or Bay 11-7082, which is an inhibitor of the TNF-a signaling pathway. This suppressive effect of DHMBA was not further enhanced in the presence of Bay 11-7082. As a mechanism underlying this effect, DHMBA was found to reduce the level of NF-kB p65 and the activity of NF-kB reporter gene expression in MC3T3-E1 cells. It has been thus found that DHMBA prevents impairment of osteoblast calcification induced by the inflammatory cytokine TNF-a through suppression of TNF-a signaling involved in macrophage activation in the bone marrow microenvironment. The present invention provides a novel strategy for the treatment of inflammation-induced osteoblast disorders.

### (Introduction)

Bone homeostasis is modulated by the functions of osteoblasts, osteocytes, and osteoclasts, which are the principal cells of bone tissue. In bone homeostasis, bone resorption is first induced by osteoclasts. These osteoclasts differentiate from hematopoietic precursor cells and develop at sites on the bone surface. Subsequently, cavities generated by osteoclastic resorption are refilled by osteoblasts that arise from local mesenchymal stem cells. Osteoblasts gather at the bottom of the cavities and initiate bone formation. Through such cellular mechanisms, bone tissue maintains a fresh and flexible structure. Bone homeostasis accompanied by this bone reconstruction (bone remodeling) mechanism plays a physiological role in maintaining new bone mass.

The process of bone remodeling is complexly regulated by hormones and cytokines; stresses acting on the skeletal system associated with physical activity and weight loading; various growth factors such as transforming growth factor-b1, platelet-derived growth factors, and insulin-like growth factors, which are produced by osteoblasts; as well as systemic influences and interactions among these factors. In addition, immune cells such as lymphocytes and macrophages present in the bone marrow microenvironment contribute to the regulation of bone remodeling.

Postmenopausal osteoporosis, which physiologically causes bone mass loss in women with aging, is induced by age-related estrogen deficiency. In addition, notably, severe bone mass loss is induced by bone-metastatic cancer cells, leading to death in patients with fractures. In particular, inflammatory macrophages contribute to the development of bone disorders mediated by bone metastasis of cancer cells. Inflammatory cytokines, mainly tumor necrosis factor (TNF)-a, cause osteoarthritis, osteoporosis, and bone metastasis of cancer. Interestingly, TNF-a produced by inflammatory macrophages has been shown to suppress osteoblast calcification through activation of NF-kB signaling and to stimulate bone resorption by osteoclasts. Macrophage RAW264.7 cells are monocyte/macrophage-like cells that have been well characterized with respect to macrophage-mediated immune, metabolic, and phagocytic functions. RAW264.7 cells are used in research as a cellular model of osteoclastogenesis in which osteoclasts differentiate from the monocyte-macrophage lineage. The host immune system is stimulated by lipopolysaccharide (LPS), which is a major antigen of Gram-negative bacteria. In the bone marrow microenvironment, inflammatory macrophages activated by LPS stimulation produce TNF-a and play a major role in bone mass loss. Clinically, development of therapeutic agents for preventing bone mass loss and fractures caused by various diseases such as inflammation and bone metastasis of cancer is important.

The marine-derived phenolic antioxidant 3,5-dihydroxy-4-methoxybenzyl alcohol (DHMBA) was first discovered in the Pacific oyster Crassostrea gigas. DHMBA has been shown to scavenge radicals in several types of cells and thereby prevent oxidative stress. Interestingly, DHMBA is an excellent peroxyl radical scavenger and exhibits approximately 15-fold and four orders of magnitude greater scavenging activity than Trolox, a known strong radical scavenger, in lipid media and aqueous media, respectively. In addition, DHMBA has been shown to react with HOO(•) more rapidly than other known antioxidants such as resveratrol and ascorbic acid.

### CITATION LIST

### PATENT DOCUMENT

Patent Document 1: JP 2023-080697 A

### DISCLOSURE OF THE INVENTION

### PROBLEMS TO BE SOLVED BY THE INVENTION

Based on the above-described properties, DHMBA is suggested to play an important role in modulation of cellular functions and maintenance of health conditions. In particular, the present inventors have demonstrated that DHMBA exerts suppressive effects on proliferation and metastatic activity of bone-metastatic prostate cancer cells. Bone-forming factors are clinically important for repairing bone mass loss and for preventing osteopenia and osteoporosis associated with aging and various diseases. Accordingly, development of such a substance is important.

In the present invention, it has been investigated whether DHMBA stimulates osteoblast calcification and prevents impairment of osteoblast calcification associated with TNF-a produced by LPS-stimulated inflammatory macrophage RAW264.7 cells. In the present invention, it has been found that DHMBA stimulates calcification of mouse osteoblast MC3T3-E1 cells, and that DHMBA also suppresses impairment of cell proliferation and calcification of osteoblast MC3T3-E1 cells mediated through signaling related to TNF-a produced by inflammatory macrophage RAW264.7 cells activated by LPS stimulation.

An object of the present invention is to provide a novel strategy for protecting against and treating impaired bone formation and calcification of osteoblasts associated with inflammatory conditions in the bone marrow microenvironment, by providing a suppressing agent or the like including 3,5-dihydroxy-4-methoxybenzyl alcohol (DHMBA) as an active ingredient.

### SOLUTION TO THE PROBLEM

The present invention is characterized by:
a calcification enhancer for osteoblast MC3T3-E1 cells, the enhancer including 3,5-dihydroxy-4-methoxybenzyl alcohol (DHMBA) as an active ingredient and having an action of enhancing calcification of osteoblast MC3T3-E1 cells;
a TNF-a effect regulator of an effect of TNF-a on proliferation or cell death of osteoblast MC3T3-E1 cells, the regulator including 3,5-dihydroxy-4-methoxybenzyl alcohol (DHMBA) as an active ingredient and having an action of regulating an effect of TNF-a on proliferation or cell death of osteoblast MC3T3-E1 cells;
a preventive agent for TNF-a-induced calcification impairment in MC3T3-E1 cells, the agent including 3,5-dihydroxy-4-methoxybenzyl alcohol (DHMBA) as an active ingredient and having an action of regulating an effect of TNF-a on proliferation or cell death of osteoblast MC3T3-E1 cells and having an action of preventing TNF-a-induced calcification impairment in the MC3T3-E1 cells;
a suppressive regulator for regulating suppression of proliferation and enhancement of cell death of osteoblasts MC3T3-E1 co-cultured with inflammatory macrophage RAW264.7 cells, the regulator including 3,5-dihydroxy-4-methoxybenzyl alcohol (DHMBA) as an active ingredient and having an action of regulating suppression of proliferation and enhancement of cell death of osteoblasts MC3T3-E1 co-cultured with inflammatory macrophage RAW264.7 cells;
a protective agent for protecting against an effect of a conditioned medium obtained by culturing inflammatory macrophage RAW264.7 cells on proliferation or cell death of osteoblast MC3T3-E1 cells, the agent including 3,5-dihydroxy-4-methoxybenzyl alcohol (DHMBA) as an active ingredient and having an action of protecting against an effect of a conditioned medium obtained by culturing inflammatory macrophage RAW264.7 cells on proliferation or cell death of osteoblast MC3T3-E1 cells; or
a suppressing agent of gene promoter activity associated with a transcription factor NF-kB in osteoblasts, the agent including 3,5-dihydroxy-4-methoxybenzyl alcohol (DHMBA) as an active ingredient and having an action of suppressing gene promoter activity associated with a transcription factor NF-kB in osteoblasts.

### EFFECTS OF INVENTION

Any of the suppressing agents including 3,5-dihydroxy-4-methoxybenzyl alcohol (DHMBA) as an active ingredient according to the present invention can provide a novel strategy for treating, for example, inflammation-induced osteoblast disorders. The suppressing agents exert excellent effects of protecting against and treating impaired bone formation and calcification of osteoblasts associated with inflammatory conditions in the bone marrow microenvironment, thus offering a novel strategy.

### BRIEF DESCRIPTION OF THE DRAWINGS

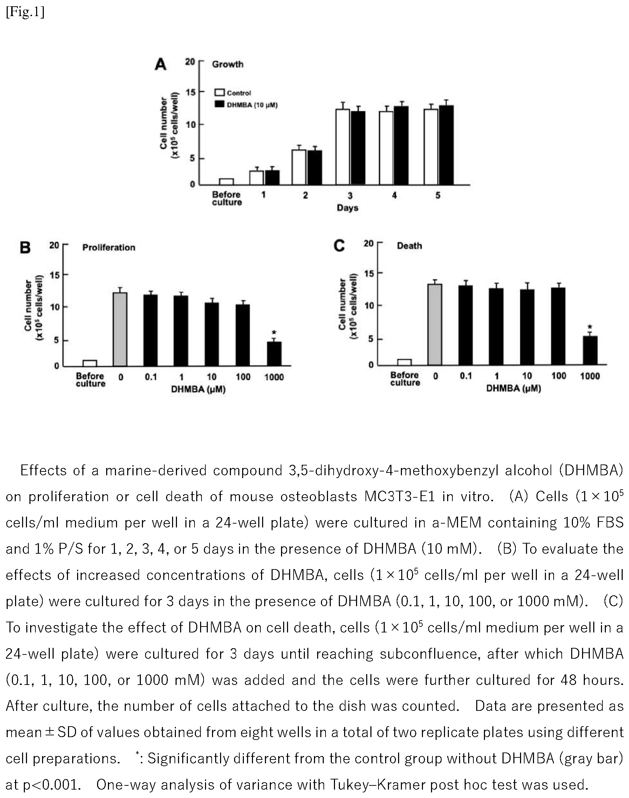

Fig. 1 is a diagram showing effects of a marine-derived compound 3,5-dihydroxy-4-methoxybenzyl alcohol (DHMBA) on proliferation or cell death of mouse osteoblasts MC3T3-E1 in vitro. (A) Cells (1×10⁵ cells/ml medium per well in a 24-well plate) were cultured in a-MEM containing 10% FBS and 1% P/S for 1, 2, 3, 4, or 5 days in the presence of DHMBA (10 mM). (B) To evaluate the effects of increased concentrations of DHMBA, cells (1×10⁵ cells/ml per well in a 24-well plate) were cultured for 3 days in the presence of DHMBA (0.1, 1, 10, 100, or 1000 mM). (C) To investigate the effect of DHMBA on cell death, cells (1×10⁵ cells/ml medium per well in a 24-well plate) were cultured for 3 days until reaching subconfluence, after which DHMBA (0.1, 1, 10, 100, or 1000 mM) was added and the cells were further cultured for 48 hours. After culture, the number of cells attached to the dish was counted. Data are presented as mean±SD of values obtained from eight wells in a total of two replicate plates using different cell preparations. *: Significantly different from the control group without DHMBA (gray bar) at p<0.001. One-way analysis of variance with Tukey-Kramer post hoc test was used.

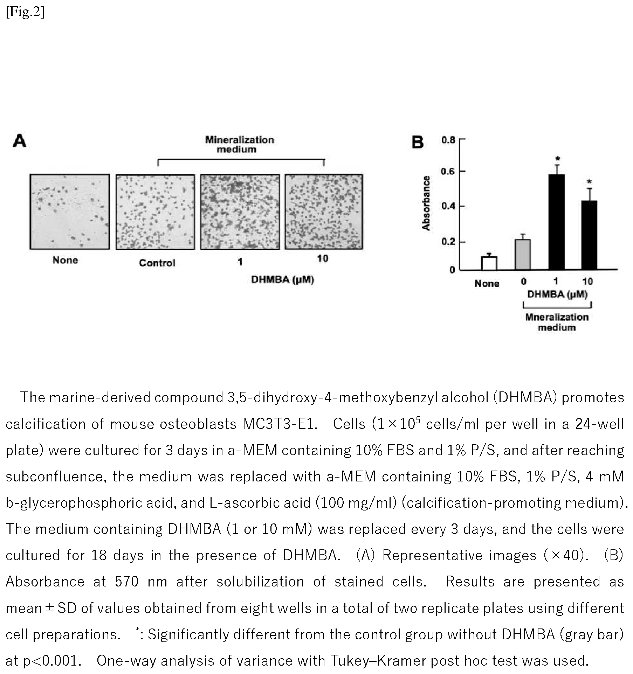

Fig. 2 is a diagram showing that the marine-derived compound 3,5-dihydroxy-4-methoxybenzyl alcohol (DHMBA) promotes calcification of mouse osteoblasts MC3T3-E1. Cells (1×10⁵ cells/ml per well in a 24-well plate) were cultured for 3 days in a-MEM containing 10% FBS and 1% P/S, and after reaching subconfluence, the medium was replaced with a-MEM containing 10% FBS, 1% P/S, 4 mM b-glycerophosphoric acid, and L-ascorbic acid (100 mg/ml) (calcification-promoting medium). The medium containing DHMBA (1 or 10 mM) was replaced every 3 days, and the cells were cultured for 18 days in the presence of DHMBA. (A) Representative images (×40). (B) Absorbance at 570 nm after solubilization of stained cells. Results are presented as mean±SD of values obtained from eight wells in a total of two replicate plates using different cell preparations. *: Significantly different from the control group without DHMBA (gray bar) at p<0.001. One-way analysis of variance with Tukey-Kramer post hoc test was used.

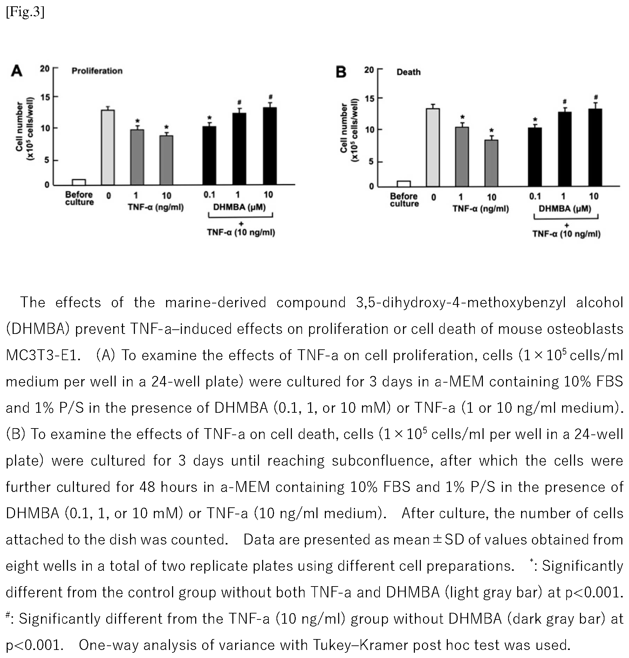

Fig. 3 is a diagram showing that the effects of the marine-derived compound 3,5-dihydroxy-4-methoxybenzyl alcohol (DHMBA) prevent TNF-a-induced effects on proliferation or cell death of mouse osteoblasts MC3T3-E1. (A) To examine the effects of TNF-a on cell proliferation, cells (1×10⁵ cells/ml medium per well in a 24-well plate) were cultured for 3 days in a-MEM containing 10% FBS and 1% P/S in the presence of DHMBA (0.1, 1, or 10 mM) or TNF-a (1 or 10 ng/ml medium). (B) To examine the effects of TNF-a on cell death, cells (1×10⁵ cells/ml per well in a 24-well plate) were cultured for 3 days until reaching subconfluence, after which the cells were further cultured for 48 hours in a-MEM containing 10% FBS and 1% P/S in the presence of DHMBA (0.1, 1, or 10 mM) or TNF-a (10 ng/ml medium). After culture, the number of cells attached to the dish was counted. Data are presented as mean±SD of values obtained from eight wells in a total of two replicate plates using different cell preparations. *: Significantly different from the control group without both TNF-a and DHMBA (light gray bar) at p<0.001. ^{#}: Significantly different from the TNF-a (10 ng/ml) group without DHMBA (dark gray bar) at p<0.001. One-way analysis of variance with Tukey-Kramer post hoc test was used.

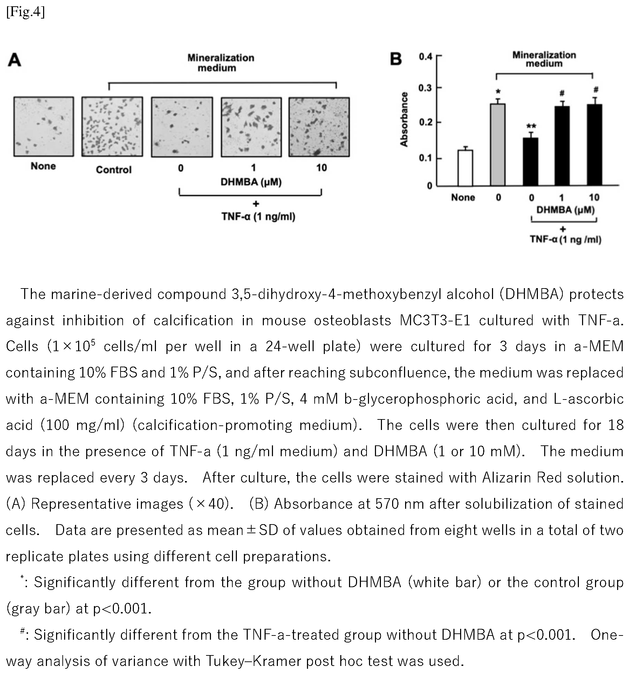

Fig. 4 is a diagram showing that the marine-derived compound 3,5-dihydroxy-4-methoxybenzyl alcohol (DHMBA) protects against inhibition of calcification in mouse osteoblasts MC3T3-E1 cultured with TNF-a. Cells (1×10⁵ cells/ml per well in a 24-well plate) were cultured for 3 days in a-MEM containing 10% FBS and 1% P/S, and after reaching subconfluence, the medium was replaced with a-MEM containing 10% FBS, 1% P/S, 4 mM b-glycerophosphoric acid, and L-ascorbic acid (100 mg/ml) (calcification-promoting medium). The cells were then cultured for 18 days in the presence of TNF-a (1 ng/ml medium) and DHMBA (1 or 10 mM). The medium was replaced every 3 days. After culture, the cells were stained with Alizarin Red solution. (A) Representative images (×40). (B) Absorbance at 570 nm after solubilization of stained cells. Data are presented as mean±SD of values obtained from eight wells in a total of two replicate plates using different cell preparations.
   *: Significantly different from the group without DHMBA (white bar) or the control group (gray bar) at p<0.001.
   ^{#}: Significantly different from the TNF-a-treated group without DHMBA at p<0.001. One-way analysis of variance with Tukey-Kramer post hoc test was used.

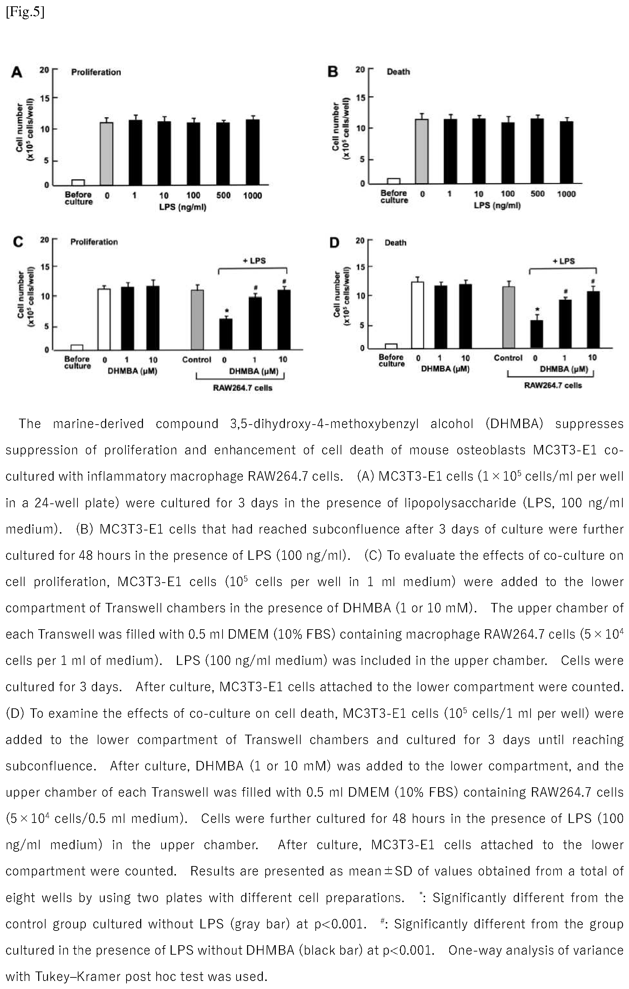

Fig. 5 is a diagram showing that the marine-derived compound 3,5-dihydroxy-4-methoxybenzyl alcohol (DHMBA) suppresses suppression of proliferation and enhancement of cell death of mouse osteoblasts MC3T3-E1 co-cultured with inflammatory macrophage RAW264.7 cells. (A) MC3T3-E1 cells (1×10⁵ cells/ml per well in a 24-well plate) were cultured for 3 days in the presence of lipopolysaccharide (LPS, 100 ng/ml medium). (B) MC3T3-E1 cells that had reached subconfluence after 3 days of culture were further cultured for 48 hours in the presence of LPS (100 ng/ml). (C) To evaluate the effects of co-culture on cell proliferation, MC3T3-E1 cells (10⁵ cells per well in 1 ml medium) were added to the lower compartment of Transwell chambers in the presence of DHMBA (1 or 10 mM). The upper chamber of each Transwell was filled with 0.5 ml DMEM (10% FBS) containing macrophage RAW264.7 cells (5×10⁴ cells per 1 ml of medium). LPS (100 ng/ml medium) was included in the upper chamber. Cells were cultured for 3 days. After culture, MC3T3-E1 cells attached to the lower compartment were counted. (D) To examine the effects of co-culture on cell death, MC3T3-E1 cells (10⁵ cells/1 ml per well) were added to the lower compartment of Transwell chambers and cultured for 3 days until reaching subconfluence. After culture, DHMBA (1 or 10 mM) was added to the lower compartment, and the upper chamber of each Transwell was filled with 0.5 ml DMEM (10% FBS) containing RAW264.7 cells (5×10⁴ cells/0.5 ml medium). Cells were further cultured for 48 hours in the presence of LPS (100 ng/ml medium) in the upper chamber. After culture, MC3T3-E1 cells attached to the lower compartment were counted. Results are presented as mean±SD of values obtained from a total of eight wells by using two plates with different cell preparations. *: Significantly different from the control group cultured without LPS (gray bar) at p<0.001. ^{#}: Significantly different from the group cultured in the presence of LPS without DHMBA (black bar) at p<0.001. One-way analysis of variance with Tukey-Kramer post hoc test was used.

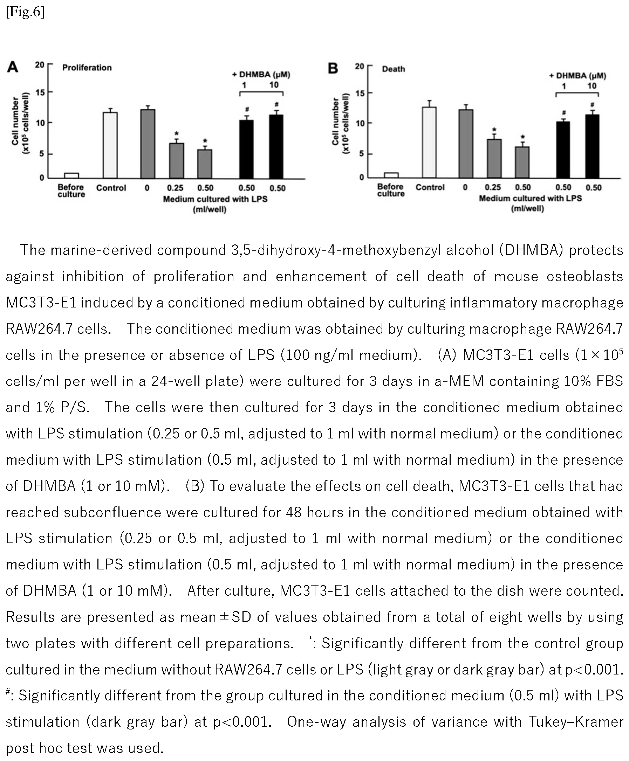

Fig. 6 is a diagram showing that the marine-derived compound 3,5-dihydroxy-4-methoxybenzyl alcohol (DHMBA) protects against inhibition of proliferation and enhancement of cell death of mouse osteoblasts MC3T3-E1 induced by a conditioned medium obtained by culturing inflammatory macrophage RAW264.7 cells. The conditioned medium was obtained by culturing macrophage RAW264.7 cells in the presence or absence of LPS (100 ng/ml medium). (A) MC3T3-E1 cells (1×10⁵ cells/ml per well in a 24-well plate) were cultured for 3 days in a-MEM containing 10% FBS and 1% P/S. The cells were then cultured for 3 days in the conditioned medium obtained with LPS stimulation (0.25 or 0.5 ml, adjusted to 1 ml with normal medium) or the conditioned medium with LPS stimulation (0.5 ml, adjusted to 1 ml with normal medium) in the presence of DHMBA (1 or 10 mM). (B) To evaluate the effects on cell death, MC3T3-E1 cells that had reached subconfluence were cultured for 48 hours in the conditioned medium obtained with LPS stimulation (0.25 or 0.5 ml, adjusted to 1 ml with normal medium) or the conditioned medium with LPS stimulation (0.5 ml, adjusted to 1 ml with normal medium) in the presence of DHMBA (1 or 10 mM). After culture, MC3T3-E1 cells attached to the dish were counted. Results are presented as mean±SD of values obtained from a total of eight wells by using two plates with different cell preparations. *: Significantly different from the control group cultured in the medium without RAW264.7 cells or LPS (light gray or dark gray bar) at p<0.001. ^{#}: Significantly different from the group cultured in the conditioned medium (0.5 ml) with LPS stimulation (dark gray bar) at p<0.001. One-way analysis of variance with Tukey-Kramer post hoc test was used.

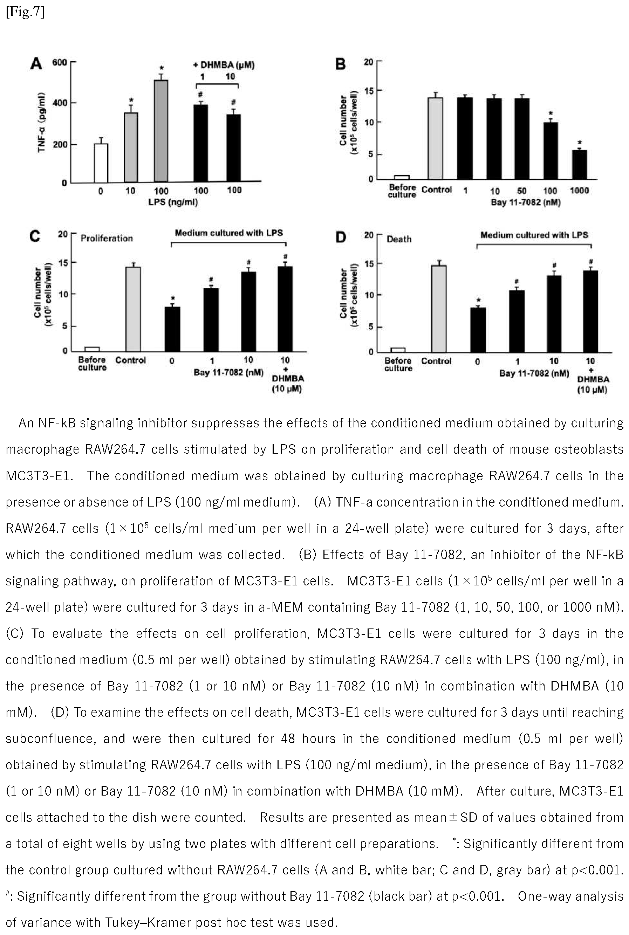

Fig. 7 is a diagram showing that an NF-kB signaling inhibitor suppresses the effects of the conditioned medium obtained by culturing macrophage RAW264.7 cells stimulated by LPS on proliferation and cell death of mouse osteoblasts MC3T3-E1. The conditioned medium was obtained by culturing macrophage RAW264.7 cells in the presence or absence of LPS (100 ng/ml medium). (A) TNF-a concentration in the conditioned medium. RAW264.7 cells (1×10⁵ cells/ml medium per well in a 24-well plate) were cultured for 3 days, after which the conditioned medium was collected. (B) Effects of Bay 11-7082, an inhibitor of the NF-kB signaling pathway, on proliferation of MC3T3-E1 cells. MC3T3-E1 cells (1×10⁵ cells/ml per well in a 24-well plate) were cultured for 3 days in a-MEM containing Bay 11-7082 (1, 10, 50, 100, or 1000 nM). (C) To evaluate the effects on cell proliferation, MC3T3-E1 cells were cultured for 3 days in the conditioned medium (0.5 ml per well) obtained by stimulating RAW264.7 cells with LPS (100 ng/ml), in the presence of Bay 11-7082 (1 or 10 nM) or Bay 11-7082 (10 nM) in combination with DHMBA (10 mM). (D) To examine the effects on cell death, MC3T3-E1 cells were cultured for 3 days until reaching subconfluence, and were then cultured for 48 hours in the conditioned medium (0.5 ml per well) obtained by stimulating RAW264.7 cells with LPS (100 ng/ml medium), in the presence of Bay 11-7082 (1 or 10 nM) or Bay 11-7082 (10 nM) in combination with DHMBA (10 mM). After culture, MC3T3-E1 cells attached to the dish were counted. Results are presented as mean±SD of values obtained from a total of eight wells by using two plates with different cell preparations. *: Significantly different from the control group cultured without RAW264.7 cells (A and B, white bar; C and D, gray bar) at p<0.001. ^{#}: Significantly different from the group without Bay 11-7082 (black bar) at p<0.001. One-way analysis of variance with Tukey-Kramer post hoc test was used.

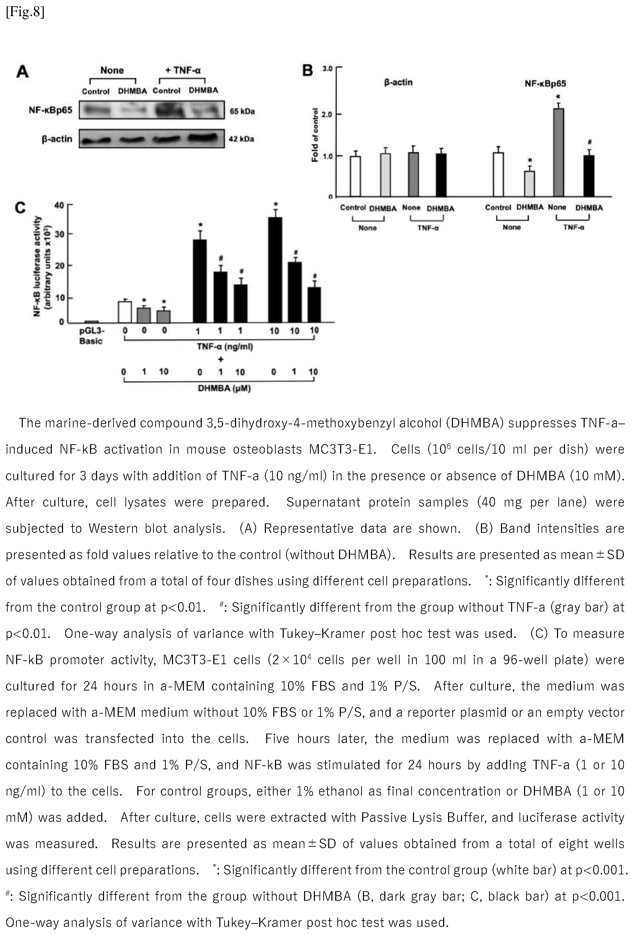

Fig. 8 is a diagram showing that the marine-derived compound 3,5-dihydroxy-4-methoxybenzyl alcohol (DHMBA) suppresses TNF-a-induced NF-kB activation in mouse osteoblasts MC3T3-E1. Cells (10⁶ cells/10 ml per dish) were cultured for 3 days with addition of TNF-a (10 ng/ml) in the presence or absence of DHMBA (10 mM). After culture, cell lysates were prepared. Supernatant protein samples (40 mg per lane) were subjected to Western blot analysis. (A) Representative data are shown. (B) Band intensities are presented as fold values relative to the control (without DHMBA). Results are presented as mean±SD of values obtained from a total of four dishes using different cell preparations. *: Significantly different from the control group at p<0.01. ^{#}: Significantly different from the group without TNF-a (gray bar) at p<0.01. One-way analysis of variance with Tukey-Kramer post hoc test was used. (C) To measure NF-kB promoter activity, MC3T3-E1 cells (2×10⁴ cells per well in 100 ml in a 96-well plate) were cultured for 24 hours in a-MEM containing 10% FBS and 1% P/S. After culture, the medium was replaced with a-MEM medium without 10% FBS or 1% P/S, and a reporter plasmid or an empty vector control was transfected into the cells. Five hours later, the medium was replaced with a-MEM containing 10% FBS and 1% P/S, and NF-kB was stimulated for 24 hours by adding TNF-a (1 or 10 ng/ml) to the cells. For control groups, either 1% ethanol as final concentration or DHMBA (1 or 10 mM) was added. After culture, cells were extracted with Passive Lysis Buffer, and luciferase activity was measured. Results are presented as mean±SD of values obtained from a total of eight wells using different cell preparations. *: Significantly different from the control group (white bar) at p<0.001. ^{#}: Significantly different from the group without DHMBA (B, dark gray bar; C, black bar) at p<0.001. One-way analysis of variance with Tukey-Kramer post hoc test was used.

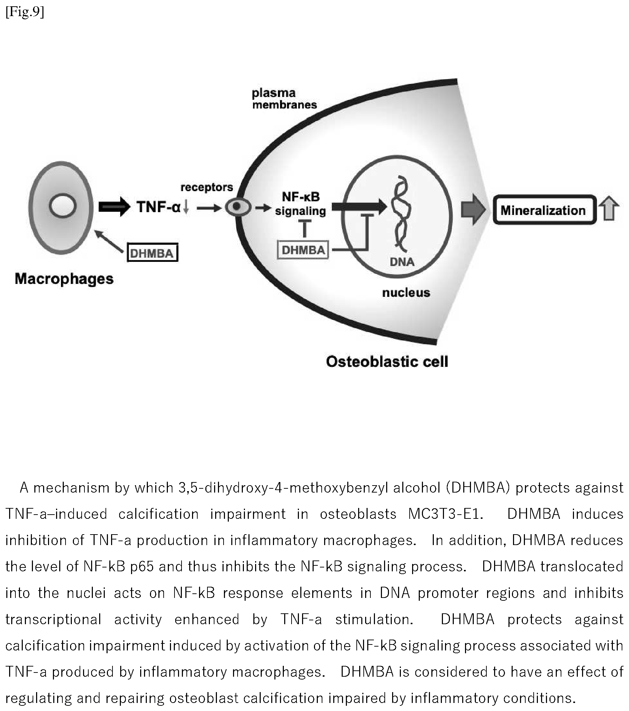

Fig. 9 is a diagram showing a mechanism by which 3,5-dihydroxy-4-methoxybenzyl alcohol (DHMBA) protects against TNF-a-induced calcification impairment in osteoblasts MC3T3-E1. DHMBA induces inhibition of TNF-a production in inflammatory macrophages. In addition, DHMBA reduces the level of NF-kB p65 and thus inhibits the NF-kB signaling process. DHMBA translocated into the nuclei acts on NF-kB response elements in DNA promoter regions and inhibits transcriptional activity enhanced by TNF-a stimulation. DHMBA protects against calcification impairment induced by activation of the NF-kB signaling process associated with TNF-a produced by inflammatory macrophages. DHMBA is considered to have an effect of regulating and repairing osteoblast calcification impaired by inflammatory conditions.

### DESCRIPTION OF EMBODIMENTS

Detailed experiments were conducted using mouse osteoblast MC3T3-E1 cells to verify the effects of various agents according to the present invention. These agents include 3,5-dihydroxy-4-methoxybenzyl alcohol (DHMBA) as an active ingredient. Details of these experiments are described below.

### (Materials and methods)

### (Reagents)

Dulbecco's Modified Eagle Medium (DMEM) containing 4.5 g/liter (1) glucose, L-glutamine, sodium pyruvate, and antibiotics (100 units/mL penicillin and 100 mg/mL streptomycin; 1% P/S) was obtained from Corning Inc. (formerly Mediatech, Inc., Manassas, VA, USA). a-Minimal Essential Medium (a-MEM) was purchased from Invitrogen Corp. (Carlsbad, CA). Fetal bovine serum (FBS) was purchased from HyClone Laboratories, Inc. (Logan, UT, USA). Lipopolysaccharide (LPS), Bay 11-7089, and all other reagents were purchased from Sigma-Aldrich (St. Louis, MO, USA), unless otherwise specified. Lipopolysaccharide (LPS) and Bay 11-7089 were dissolved in 100% ethanol and stored at -20°C until use.

### (3,5-dihydroxy-4-methoxybenzyl alcohol)

3,5-dihydroxy-4-methoxybenzyl alcohol (hereinafter abbreviated as DHMBA), which is a novel amphiphilic phenolic compound, was first isolated from the Pacific oyster (Crassostrea gigas) as an antioxidant. In the present invention, synthetically prepared DHMBA was used. The structure of DHMBA has been reported in previous studies. The purity of the synthesized DHMBA was 100%. DHMBA was dissolved in 100% ethanol and stored at -20°C until use.

### (Osteoblast MC3T3-E1 cells)

Mouse osteoblast MC3T3-E1 cells were obtained from the American Type Culture Collection (HTB-77TM, ATCC; Rockville, MD, USA). MC3T3-E1 cells were cultured, as previously used, in a-MEM containing 10% FBS and 1% P/S at 37°C in a CO₂ incubator using plastic dishes.

### (Macrophage RAW264.7 cells)

Mouse RAW264.7 cells were purchased from the American Type Culture Collection (Rockville, MD, USA), as previously described. RAW264.7 cells are a monocyte/macrophage-like cell line derived from a cell line transformed with Abelson leukemia virus originating from BALB/c mice.

### (Cell proliferation assay)

To examine progression of cell proliferation, MC3T3-E1 cells (1×10⁵/ml medium per well) were cultured using a 24-well plate in a-MEM supplemented with 10% FBS and 1% P/S at 37°C in a water-saturated atmosphere containing 5% CO₂ and 95% air. The cells were cultured for 1, 2, 3, 4, or 5 days in the presence of a vehicle (1% ethanol as final concentration) or DHMBA (10 mM). In another experiment, to evaluate the effects of increased concentrations of DHMBA on MC3T3-E1 cells, the cells (1×10⁵/ml per well) were cultured using a 24-well plate in DMEM (containing 10% FBS and 1% P/S). The cells were cultured for 3 days in the presence of either a reagent vehicle (1% ethanol as final concentration) or DHMBA (0.1, 1, 10, 100, or 1000 mM).

To evaluate the effects of DHMBA on cell death of osteoblasts MC3T3-E1, the cells (1×10⁵/ml medium per well in a 24-well plate) were cultured in a-MEM containing 10% FBS and 1% P/S. After culturing for 3 days until reaching subconfluence, either a reagent vehicle (1% ethanol as final concentration) or DHMBA (0.1, 1, 10, 100, or 1000 mM) was added, and the cells were further cultured for 48 hours. After culture, to count the number of cells attached to each well, a Ca²⁺/Mg²⁺ solution containing 0.05% trypsin and EDTA (0.1 ml per well) was added, and then the culture plates were incubated at 37°C for 2 minutes. Subsequently, a-MEM (0.9 ml) containing 10% FBS and 1% P/S was added, and the cells were detached and mixed by pipetting. The cell suspension (0.1 ml) was mixed with 0.1 ml of 0.5% trypan blue staining solution. By this staining, viable cells are stained, whereas dead cells are not stained. Viable cells were counted under a microscope (Olympus MTV-3) using a hemocytometer (Sigma-Aldrich, St. Louis, MO) and a cell counter (H-102P, Line Seiki Co., Ltd., Tokyo, Japan), and the average of two counts was calculated. Cell numbers were expressed as the number per well.

### (Osteoblast differentiation assay and Alizarin Red S staining)

MC3T3-E1 cells (10⁵ cells/ml medium per well in a 12-well plate) were cultured for 3 days in a-MEM (1.0 ml per well) containing 10% FBS and 1% P/S. Thereafter, the medium was replaced, as previously described, with calcification-promoting medium, that is, a-MEM supplemented with 10% FBS, 1% P/S, L-ascorbic acid (100 mg/ml), and 4 mM b- glycerophosphoric acid. DHMBA was added at a concentration of 1 or 10 mM, and the cells were cultured for 18 days, with the medium replaced every 3 days with fresh medium. On day 18 of culture, the cells were rinsed with PBS and fixed by addition of ice-cold 75% ethanol at 4°C for 30 minutes. Then, calcium deposition was visualized by staining with Alizarin Red S (40 mM, pH 6.2). After incubation at room temperature for 30 minutes, the staining solution was removed by thorough washing with distilled water. Stained cells on the plates were photographed under a microscope (×40) (Olympus IX71, Olympus Corp., Tokyo, Japan) and imaged using ImageJ2 software. For quantification, the stained cells in each plate were solubilized by addition of 10% cetylpyridinium chloride solution, and absorbance was measured at 570 nm.

### (Crosstalk between MC3T3-E1 cells and RAW264.7 cells)

To investigate crosstalk between osteoblast MC3T3-E1 cells and macrophage RAW264.7 cells, an assay was performed using Transwell chambers (Corning Inc., Life Sciences, catalog no. CLS3464-48EA, USA) equipped with a polycarbonate filter (pore size 8 mm, diameter 6.5 mm). MC3T3-E1 cells (1×10⁵ cells per 1 ml medium) were added in a-MEM medium (1 ml) containing 10% FBS and 1% P/S in the lower chamber. RAW264.7 cells were suspended in DMEM containing 10% FBS and 1% P/S, and 0.5 ml of the suspension (5×10⁴ cells/ml medium) was added to the upper chamber of each Transwell. RAW264.7 cells were incubated at 37°C for 1 hour under 5% CO₂ and 95% air to allow attachment to the upper chamber. The plates equipped with the Transwell chambers were then placed in an incubator at 37°C under 5% CO₂ and 95% air. To induce inflammatory conditions in RAW264.7 cells, LPS (100 ng/ml medium) was added to the upper chamber containing RAW264.7 cells. The culture plates containing MC3T3-E1 (lower compartment) and RAW264.7 cells (upper compartment) were cultured for 3 days until reaching subconfluence. After culture, the media in the upper chamber and lower compartment were aspirated, and the filters were removed. MC3T3-E1 cells attached to the dishes (wells) were counted as described in the section "Cell proliferation assay".

Further, to examine the effects of co-culture with RAW264.7 cells on cell death of MC3T3-E1 cells, osteoblasts (1×10⁵ cells/ml per well) were cultured for 3 days until reaching subconfluence, after which the medium was replaced with fresh medium. This medium contained either a reagent vehicle (1% ethanol as final concentration) or DHMBA (1 or 10 mM).

Thereafter, a Transwell chamber was placed in each culture well, and RAW264.7 cells were added to the upper chamber at 0.5 ml (5×10⁴ cells/ml medium), in the presence or absence of LPS (100 ng/ml medium). After a further 48 hours of culture, the media in the upper chamber and lower compartment were aspirated and the filters were removed. Cells attached to the dishes (wells) were counted as described in the section "Cell proliferation assay".

### (Effects of conditioned medium on MC3T3-E1 cells)

A conditioned medium was obtained by culturing RAW264.7 cells.

RAW264.7 cells (1×10⁵ cells/ml per well in a 24-well plate) were cultured for 3 days after reaching subconfluence in DMEM containing 10% FBS and 1% P/S in the presence or absence of LPS (concentration in the medium: 100 ng/ml). After the cells were cultured, the medium (conditioned medium) was collected.

To evaluate the effects of the conditioned medium on proliferation of MC3T3-E1 cells, MC3T3-E1 cells were cultured for 3 days in a-MEM containing 10% FBS and 1% P/S. The cells were then cultured for 3 days using either the conditioned medium (1 ml, control) cultured without LPS or the conditioned medium (0.25 or 0.50 ml per well, adjusted to 1 ml with normal medium) cultured with LPS in the presence or absence of DHMBA (1 or 10 mM).

Further, to examine the effects on cell death of MC3T3-E1 cells, the cells (1×10⁵ cells/ml per well) were cultured for 3 days in a-MEM containing 10% FBS and 1% P/S. After reaching subconfluence, the medium was replaced. The cells were then further cultured for 48 hours in either the normal medium, the conditioned medium (1 ml) cultured without LPS, or the conditioned medium (0.25 or 0.50 ml per well, adjusted to 1 ml with normal medium) with either a reagent vehicle (1% ethanol as final concentration) or DHMBA (1 or 10 mM). After culture, the cells were detached from each culture well, and cell numbers were counted in the same manner as described above.

### (TNF-a production assay)

RAW264.7 cells (1× 10⁵/ml per well) were cultured in DMEM containing 10% FBS and 1% P/S in a 24-well plate for 3 days until reaching subconfluence. The cells were treated with or without LPS (100 ng/ml medium) and incubated for 5 hours, and then the medium was collected for cytokine assay. The cells were detached from each culture dish to count the cell numbers as described in the section "Cell proliferation assay". No significant change in the number of RAW264.7 cells was observed under the LPS treatment. The concentration of TNF-a in the medium was analyzed using a mouse TNF-a ELISA kit (catalog no. KHC301) purchased from Cayman Chemical Company (Ann Arbor, MI, USA) in accordance with the manufacturer's instructions. TNF-a production was expressed as the amount of TNF-a secreted into the medium in picograms per milliliter (pg/ml).

### (Effects of NF-kB signaling pathway inhibitor)

Further, the effects of Bay 11-7082, which is an NF-kB signaling inhibitor, on proliferation or cell death of MC3T3-E1 cells were investigated. First, to examine the effects of Bay 11-7082 on proliferation of MC3T3-E1 cells, the cells (1×10⁵ cells/1 ml per well in a 24-well plate) were cultured for 3 days until reaching subconfluence in the presence of 10% FBS, 1% P/S, and either a reagent vehicle (1% ethanol as final concentration) or Bay 11-7082 (1, 10, 50, 100, or 1000 nM). Next, MC3T3-E1 cells were cultured for 3 days in the presence of the conditioned medium (0.5 ml per well, adjusted to a total of 1 ml with normal medium) with or without Bay 11-7082 (1 or 10 nM) in the presence or absence of DHMBA (10 mM).

In another experiment, to evaluate the effects of Bay 11-7082 on cell death of MC3T3-E1, the cells (1×10⁵ cells/1 ml per well in a 24-well plate) were cultured in a-MEM containing 10% FBS and 1% P/S. After culturing for 3 days until reaching subconfluence, the medium was replaced with a-MEM (1 ml) containing either the normal medium or the conditioned medium prepared by adding the normal medium to 0.5 ml per well of the conditioned medium, adjusted to a total volume of 1 ml. The cells were then further cultured for 48 hours in the presence of Bay 11-7082 (1 or 10 nM), or Bay 11-7082 (10 nM) in combination with DHMBA (10 mM). After culture, the cells were detached from each culture well, and cell numbers were counted as described above.

### (Western blotting)

MC3T3-E1 cells (1×10⁶ cells in 10 ml medium in a 100-mm dish) were cultured for 3 days in a-MEM containing 10% FBS and 1% P/S in the presence of a reagent vehicle (1% ethanol as final concentration), DHMBA (10 mM), TNF-a (10 ng/ml medium), or DHMBA (10 mM) in combination with TNF-a (10 ng/ml). After culture, the dishes were washed three times with cold PBS (10 ml), and adherent cells were removed from the dishes by scraping in Cell Lysis Buffer (Cell Signaling Technology, Inc., Danvers, MA, USA). Protein samples obtained from the cell lysates were loaded (40 mg per lane) and separated by SDS-polyacrylamide gel electrophoresis (12% SDS-PAGE), as previously described. After electrophoresis, the membranes were incubated overnight at 4°C with primary antibodies (each diluted 1:1,000) against target proteins NF-kB p65 (catalog no. 3034, rabbit) and b-actin (catalog no. 3700, mouse). After incubation, the membranes were further incubated at room temperature for 60 minutes with horseradish peroxidase-conjugated secondary antibodies (Santa Cruz Biotechnology Inc., mouse sc-2005 or rabbit sc-2305, each diluted 1:1,000). Protein bands were then detected using a chemiluminescent substrate (catalog no. 34577, Thermo Fisher Scientific Inc., Rockford, IL, USA) on X-ray film. A total of four films obtained from four independent experiments using separate membranes were scanned using an Epson Perfection 1660 Photo scanner, and band intensities were quantified using ImageJ2 software (National Institutes of Health, Bethesda, MD, USA).

### (NF-kB reporter assay)

An NF-kB-responsive luciferase reporter assay (pNF-kB-Luc, BD Biosciences) was performed according to the method previously described by the present inventors. MC3T3-E1 cells (2×10⁴ cells per well in 100 ml in a 96-well plate) were cultured for 24 hours in a-MEM containing 10% FBS and 1% P/S. After culture, the medium was removed, and the cells were washed twice with a-MEM without FBS or antibiotics. a-MEM (100 ml) without FBS or antibiotics was then added to each well containing the cells. A reporter plasmid or an empty vector control (pGL3-Basic) was transfected into MC3T3-E1 cells using Lipofectamine 2000 Reagent (Invitrogen) in a-MEM without FBS or antibiotics. Five hours later, the medium was replaced with a-MEM containing 10% FBS and 1% P/S, and the cells were stimulated with TNF-a (1 or 10 ng/ml) for 24 hours. In parallel, the cells were cultured with either a reagent vehicle (1% ethanol as final concentration) or DHMBA (1 or 10 mM). After culture, the cells were extracted with Passive Lysis Buffer (Promega Corp., Madison, WI), and luciferase activity was measured using a Luciferase Assay System (Promega Corp.) with a microplate reader (Turner Designs, Inc., Sunnyvale, CA, USA). Equal transfection efficiency among all plasmids was validated by using the luciferase reporter plasmid pRL-SV40 (Promega Corp., Madison, WI).

### (Statistical analysis)

Statistical significance was determined using GraphPad InStat version 3 for Windows XP (GraphPad Software, Inc., La Jolla, CA). Data are presented as mean±standard deviation (SD). Multiple comparisons were performed by one-way analysis of variance (ANOVA) with Tukey-Kramer multiple comparison post hoc test for parametric data. A p-value of <0.05 was considered statistically significant.

### (Results)

### Effects of marine-derived compound 3,5-dihydroxy-4-methoxybenzyl alcohol (DHMBA) on proliferation and cell death of osteoblast MC3T3-E1 cells

To elucidate the effects of DHMBA on proliferation and cell death of mouse osteoblasts MC3T3-E1, the cells (1×10⁵ cells/ml medium per well in a 24-well plate) were cultured for 1, 2, 3, 4, or 5 days in a-MEM medium containing 10% FBS and 1% P/S in the presence of a reagent vehicle (1% ethanol as final concentration) or DHMBA (10 mM). After 5 days of culture, no significant change in proliferation of MC3T3-E1 cells was observed in vitro (Fig. 1A).

To examine the effects of increased concentrations of DHMBA, the cells (1×10⁵ cells/ml per well) were cultured for 3 days in the presence of a reagent vehicle (1% ethanol as final concentration) or DHMBA (0.1, 1, 10, 100, or 1000 mM). Proliferation of MC3T3-E1 cells was not changed by culture with DHMBA (0.1, 1, 10, or 100 mM) (Fig. 1B). However, at the highest concentration tested (1000 mM), DHMBA induced a significant decrease in cell proliferation.

Further, to evaluate the effects of DHMBA on cell death, the cells (1×10⁵ cells/ml per well) were cultured for 3 days until reaching subconfluence, after which DHMBA (0.1, 1, 10, 100, or 1000 mM) was added and the cells were further cultured for 48 hours. The number of cells attached to the dishes was not changed by addition of DHMBA (0.1, 1, 10, or 100 mM). However, at the highest concentration of DHMBA tested (1000 mM), the number of cells was significantly decreased (Fig. 1C). Thus, it has been found that culture with relatively low concentrations of DHMBA does not cause changes in the number of MC3T3-E1 cells.

### (DHMBA stimulates calcification of osteoblast MC3T3-E1 cells)

MC3T3-E1 cells (1×10⁵ cells/ml per well) were cultured for 3 days in a-MEM containing 10% FBS and 1% P/S, and after reaching subconfluence, the medium was replaced with the calcification-promoting medium containing 10% FBS, 1% P/S, 4 mM b- glycerophosphoric acid, and L-ascorbic acid (100 mg/ml). This medium was replaced every 3 days with fresh medium. The cells were cultured for 18 days in the medium with either a reagent vehicle (1% ethanol as final concentration) or DHMBA (1 or 10 mM). After culture, the cells were stained with Alizarin Red solution. Culture with DHMBA (1 or 10 mM) enhanced calcification of MC3T3-E1 cells (Figs. 2A and 2B). These results suggest that culture with DHMBA enhances calcification of osteoblast MC3T3-El cells in vitro.

### (DHMBA regulates effects of TNF-a on proliferation or cell death of osteoblast MC3T3-E1 cells)

TNF-a has been shown to inhibit bone formation and calcification in osteoblasts. Accordingly, it was investigated whether DHMBA modulated the effects of TNF-a on osteoblasts in vitro. To evaluate the effects of TNF-a on cell proliferation, the cells (1×10⁵ cells/ml per well) were cultured for 3 days in a-MEM containing 10% FBS in the presence of TNF-a (1 or 10 ng/ml per 1 ml medium). Culture of the cells with TNF-a (1 or 10 ng/ml) inhibited cell proliferation (Fig. 3A). This inhibition was suppressed by the presence of DHMBA (1 or 10 mM) in the medium (Fig. 3A).

Further, to examine the effects on cell death, the cells (1×10⁵ cells/ml per well) were cultured for 3 days until reaching subconfluence. The cells were then cultured for 48 hours in the medium containing TNF-a (1 or 10 ng/ml medium) in combination with DHMBA (0.1, 1, or 10 mM). Culture in the presence of TNF-a (1 or 10 ng/ml) enhanced death of MC3T3-E1 cells (Fig. 3B). This effect was suppressed by the presence of DHMBA (1 or 10 mM) (Fig. 3B). Thus, it has been found that DHMBA suppresses TNF-a-induced suppression of proliferation and stimulation of cell death in MC3T3-E1 cells.

Next, it was examined whether DHMBA prevented calcification impairment in osteoblast MC3T3-E1 cells cultured with TNF-a. The cells (1×10⁵ cells/ml per well) were cultured for 3 days in a-MEM containing 10% FBS and 1% P/S, and after reaching subconfluence, the cells were cultured for 18 days in a-MEM containing 10% FBS, 1% P/S, 4 mM b- glycerophosphoric acid, and L-ascorbic acid (100 mg/ml) (calcification-promoting medium). This medium was replaced every 3 days with fresh medium. After culture, the cells were stained with Alizarin Red solution. Calcification of osteoblasts was suppressed when the cells were cultured in the presence of TNF-a (1 ng/ml) (Fig. 4). This suppression was suppressed by the presence of DHMBA (1 or 10 mM) (Fig. 4). Thus, it has been found that DHMBA prevents TNF-a-induced calcification impairment in MC3T3-E1 cells in vitro.

### (DHMBA regulates suppression of proliferation and enhancement of cell death of osteoblasts MC3T3-E1 co-cultured with inflammatory macrophage RAW264.7 cells)

Inflammatory macrophages that produce the inflammatory cytokine TNF-a may play a pathophysiological role in the bone marrow environment. Thus, it was examined whether inflammatory macrophages affected proliferation and death of osteoblasts through crosstalk between osteoblast MC3T3-E1 cells and mouse macrophage RAW264.7 cells (Fig. 5). First, the effects of lipopolysaccharide (LPS) on the number of MC3T3-E1 cells were examined (Figs. 5A and 5B). MC3T3-E1 cells (1×10⁵ cells/ml per well) were cultured for 3 days in a-MEM containing 10% FBS in the presence or absence of LPS (1 to 1000 ng/ml in the medium). LPS did not affect proliferation of MC3T3-E1 cells (Fig. 5A). Next, to evaluate the effects of LPS on cell death, MC3T3-E1 cells that had reached subconfluence were further cultured for 48 hours in the presence of LPS (1 to 1000 ng/ml). LPS did not induce cell death (Fig. 5B).

Next, it was examined whether proliferation and cell death of MC3T3-E1 cells were affected by crosstalk between mouse macrophage RAW264.7 cells and MC3T3-E1 cells (Figs. 5C and 5D). MC3T3-E1 cells were added to the lower compartment of Transwell chambers. The upper chamber of each Transwell was filled with RAW264.7 cells containing LPS (100 ng/ml), followed by culture for 3 days. After culture, MC3T3-E1 cells attached to the lower compartment were counted. It was found that co-culture with RAW264.7 cells suppressed proliferation of MC3T3-E1 cells (Fig. 5C). This decrease was suppressed by the presence of DHMBA (1 or 10 mM).

Further, to examine the effects of co-culture on cell death, MC3T3-E1 cells were added to the lower compartment of Transwell chambers and cultured for 3 days until reaching subconfluence. After culture, DHMBA (1 or 10 mM) was added to the lower compartment, and the upper chamber of each Transwell was filled with RAW264.7 cells, followed by culture for 48 hours in the presence of LPS (100 ng/ml medium) (Fig. 5D). Co-culture with RAW264.7 cells enhanced death of MC3T3-E1 cells (Fig. 5D). This enhancement was prevented by the presence of DHMBA (1 or 10 mM) (Fig. 5D).

These results suggest that inflammatory macrophages suppress proliferation, stimulate cell death, and thereby lead to a reduction in cell number of osteoblasts. Thus, it has been found that DHMBA suppresses the effects of the macrophages on osteoblasts in vitro.

### (DHMBA protects against effects of conditioned medium obtained by culturing inflammatory macrophage RAW264.7 cells)

A conditioned medium was obtained by culturing macrophage RAW264.7 cells in the presence or absence of LPS (100 ng/ml medium). MC3T3-E1 cells (1×10⁵ cells/ml medium per well) were cultured for 3 days in a-MEM containing 10% FBS and 1% P/S, and after reaching subconfluence, the medium was replaced with the conditioned medium (0.25 or 0.50 ml, adjusted to 1 ml with normal medium) (Fig. 6A). Proliferation of MC3T3-E1 cells was inhibited by the presence of the conditioned medium. Notably, this inhibition was prevented in the presence of DHMBA (1 or 10 mM) (Fig. 6A).

Further, to evaluate the effects on cell death, MC3T3-E1 cells that had reached subconfluence were further cultured for 48 hours in the conditioned medium (0.25 or 0.50 ml, adjusted to 1 ml with normal medium) or in the conditioned medium (0.5 ml) supplemented with DHMBA (1 or 10 mM) (Fig. 6B). Death of MC3T3-E1 cells was enhanced by culture with the conditioned medium (Fig. 6B). This enhancement was suppressed when the conditioned medium contained DHMBA (1 or 10 mM) (Fig. 6B). Thus, it has been found that the conditioned medium cultured with LPS inhibits proliferation, enhances cell death, and results in a reduction in cell number of MC3T3-E1 cells.

### (Involvement of NF-kB signaling in osteoblasts MC3T3-E1 cultured with conditioned medium)

It has been demonstrated that osteoblast calcification is suppressed by activation of TNF-a-induced NF-kB signaling. Thus, it was examined whether TNF-a and its signaling process were involved in the manifestation of the effects of the conditioned medium on inhibition of proliferation and enhancement of cell death of MC3T3-E1 cells by inhibiting NF-kB signaling. Culturing with LPS (10 or 100 ng/ml) increased the production of TNF-a in RAW264.7 cells, and this increase was suppressed by the presence of DHMBA (1 or 10 mM) (Fig. 7A).

Culturing with Bay 11-7082 (1, 10, or 50 nM), which is an inhibitor of NF-kB signaling, did not cause a significant reduction in the number of MC3T3-E1 cells (Fig. 7B). Culturing with Bay 11-7082 (1 or 10 nM) suppressed the effects of the conditioned medium on the occurrence of inhibition of proliferation (Fig. 7C) and enhancement of cell death (Fig. 7D) of MC3T3-E1 cells. These results suggest that TNF-a and its NF-kB signaling process are involved in the manifestation of the effects of the conditioned medium on inhibition of proliferation and enhancement of cell death of osteoblasts MC3T3-E1.

Further, it was elucidated whether DHMBA modulated the NF-kB signaling process in MC3T3-E1 cells. Culturing with DHMBA (10 mM) reduced the expression level of NF-kB p65 in MC3T3-E1 cells (Figs. 8A and 8B). The level of NF-kB p65 was increased by culturing in the presence of TNF-a (10 ng/ml) (Fig. 8A and 8B). This increase was suppressed by culturing with DHMBA (10 mM) (Fig. 8A and 8B). In addition, NF-kB promoter activity in MC3T3-E1 cells was increased by the treatment with TNF-a (1 or 10 ng/ml) (Fig. 8C). This increase was suppressed by addition of DHMBA (1 or 10 mM) (Fig. 8C). Thus, it has been found that enhancement of NF-kB promoter activity induced by TNF-a stimulation in MC3T3-E1 cells is suppressed in the presence of DHMBA. Further, DHMBA also reduced NF-kB promoter activity in MC3T3-E1 cells in the absence of TNF-a (Fig. 8C). These results support the view that DHMBA suppresses gene promoter activity associated with the transcription factor NF-kB in osteoblasts.

### (Discussion)

The marine-derived phenolic antioxidant 3,5-dihydroxy-4-methoxybenzyl alcohol (DHMBA) was discovered as an excellent peroxyl radical scavenger. Its scavenging capacity has been shown to be approximately 15-fold and four orders of magnitude greater in lipid media and aqueous media than that of Trolox, suggesting that DHMBA plays an important role in the modulation of cellular functions and the maintenance of health conditions.

In recent years, it has been demonstrated that DHMBA exerts suppressive effects on proliferation and metastatic activity of bone-metastatic prostate cancer cells, suggesting that DHMBA may play a pharmacological role in prevention of bone mass loss. Further, in the present invention, it has been found that DHMBA stimulates osteoblast calcification and prevents impairment of osteoblast calcification associated with signaling of TNF-a produced by LPS-stimulated inflammatory macrophage RAW264.7 cells. DHMBA is therefore considered to play a pharmacologically important role in repairing impaired calcification.

Factors that enhance bone formation are clinically important for preventing and repairing loss of bone mass in osteoporosis caused by aging and various diseases. Accordingly, development of such factors is required. When osteoblasts MC3T3-E1 were cultured with DHMBA in vitro, osteoblast calcification was found to be enhanced. DHMBA exerts promotive effects on osteoblast calcification and differentiation, suggesting that DHMBA may contribute to promotion of bone formation, and is therefore considered to be important as a bone formation-promoting factor.

It is well known that bone mass loss is caused by postmenopausal estrogen deficiency associated with inflammatory conditions. The cytokine TNF-a, which is generated during inflammation, plays an important role in skeletal pathology and induces bone mass loss. This cytokine inhibits osteoblast calcification through activation of NF-kB signaling in osteoblast MC3T3-E1 cells.

In the present invention, it has been investigated whether culturing with DHMBA prevents impairment of osteoblast calcification associated with NF-kB signaling in MC3T3-E1 cells. Calcification of MC3T3-E1 cells was impaired by culturing with TNF-a that activated NF-kB signaling. This impairment was found to be suppressed by the presence of DHMBA. DHMBA has been thus found to exert an inhibitory effect on the NF-kB signaling process.

Inflammatory macrophages may play a pathophysiological role in the bone marrow microenvironment by increasing cytokine production. In addition, crosstalk between osteoblasts and macrophage RAW264.7 cells has been investigated in vitro.

When MC3T3-E1 cells were co-cultured with RAW264.7 cells under LPS stimulation, proliferation of osteoblast MC3T3-E1 cells was suppressed. This suppression of proliferation was induced by culturing osteoblasts with the conditioned medium obtained by culturing RAW264.7 cells subjected to LPS stimulation. Notably, the suppressive effects of the conditioned medium on proliferation of MC3T3-E1 cells was suppressed in the presence of an NF-kB signaling inhibitor, suggesting involvement of NF-kB signaling.

Further, the concentration of TNF-a in the conditioned medium was markedly increased when RAW264.7 cells were cultured under LPS stimulation. These results support the view that osteoblast calcification is impaired by activation of the signaling process of TNF-a produced by inflammatory macrophages in the bone marrow microenvironment. Notably, when cultured with DHMBA, production of TNF-a in inflammatory RAW264.7 cells was suppressed. In addition, the suppressive effects of the conditioned medium on cell proliferation were not observed in the medium with DHMBA, suggesting that this substance suppresses the TNF-a signaling process in MC3T3-E1 cells. Thus, DHMBA may be useful for prevention of osteoblast calcification impairment associated with inflammatory conditions.

With respect to the underlying mechanism, it was further investigated whether DHMBA affected the NF-kB signaling process in MC3T3-E1 cells. When cultured in the presence of DHMBA, the level of NF-kB p65 in osteoblasts was decreased. In addition, DHMBA was found to suppress the enhancement of reporter activity of NF-kB response elements in nuclear gene promoter regions induced by TNF-a stimulation in MC3T3-E1 cells. This mechanism is considered to be fundamentally important for suppression of TNF-a-induced impairment of osteoblast calcification by DHMBA.

As summarized in Fig. 9, inflammatory macrophages in the bone marrow microenvironment produce TNF-a, and this cytokine activates the NF-kB signaling process in osteoblasts. DHMBA suppresses production of TNF-a in inflammatory macrophages and regulates activation of the associated signaling process. Further, DHMBA may be translocated into the nucleus of osteoblast. DHMBA that is translocated into the nucleus may inhibit the binding of NF-kB to the response elements within the gene promoter regions. In addition, DHMBA may exert direct modulatory effects on transcriptional activity at the promoter regions within the nucleus.

In conclusion, in the present invention, it has been confirmed that DHMBA stimulates calcification of mouse osteoblast MC3T3-E1 cells in vitro. Further, it has been found that DHMBA regulates the occurrence of osteoblast calcification impairment that is caused through activation of the signaling process of TNF-a produced by inflammatory macrophages in vitro. Thus, DHMBA is considered to exert bone-forming effects by preventing impairment of osteoblast calcification.

The marine-derived compound DHMBA is therefore considered to play a role in prevention and repair of bone mass loss associated with inflammation. This study provides a novel strategy for treatment of impaired osteoblast bone formation associated with inflammatory conditions in the bone marrow microenvironment.

## Claims

1. A calcification enhancer for osteoblast MC3T3-E1 cells, the enhancer comprising 3,5-dihydroxy-4-methoxybenzyl alcohol (DHMBA) as an active ingredient and having an action of enhancing calcification of osteoblast MC3T3-E1 cells.

2. A TNF-a effect regulator of an effect of TNF-a on proliferation or cell death of osteoblast MC3T3-E1 cells, the regulator comprising 3,5-dihydroxy-4-methoxybenzyl alcohol (DHMBA) as an active ingredient and having an action of regulating an effect of TNF-a on proliferation or cell death of osteoblast MC3T3-E1 cells.

3. A preventive agent for TNF-a-induced calcification impairment in MC3T3-E1 cells, the agent comprising 3,5-dihydroxy-4-methoxybenzyl alcohol (DHMBA) as an active ingredient and having an action of regulating an effect of TNF-a on proliferation or cell death of osteoblast MC3T3-E1 cells and having an action of preventing TNF-a-induced calcification impairment in the MC3T3-E1 cells.

4. A suppressive regulator for regulating suppression of proliferation and enhancement of cell death of osteoblasts MC3T3-E1 co-cultured with inflammatory macrophage RAW264.7 cells, the regulator comprising 3,5-dihydroxy-4-methoxybenzyl alcohol (DHMBA) as an active ingredient and having an action of regulating suppression of proliferation and enhancement of cell death of osteoblasts MC3T3-E1 co-cultured with inflammatory macrophage RAW264.7 cells.

5. A protective agent for protecting against an effect of a conditioned medium obtained by culturing inflammatory macrophage RAW264.7 cells on proliferation or cell death of osteoblast MC3T3-E1 cells, the agent comprising 3,5-dihydroxy-4-methoxybenzyl alcohol (DHMBA) as an active ingredient and having an action of protecting against an effect of a conditioned medium obtained by culturing inflammatory macrophage RAW264.7 cells on proliferation or cell death of osteoblast MC3T3-E1 cells.

6. A suppressive agent of gene promoter activity associated with a transcription factor NF-kB in osteoblasts, the agent comprising 3,5-dihydroxy-4-methoxybenzyl alcohol (DHMBA) as an active ingredient and having an action of suppressing gene promoter activity associated with a transcription factor NF-kB in osteoblasts.
